# EUROPÄISCHE PATENTSCHRIFT

(11) **EP 2 137 489 B1**
(45) Veröffentlichungstag und Bekanntmachung des Hinweises auf die Patenterteilung: **29.12.2010**
(21) Anmeldenummer: 08708130.3
(22) Anmeldetag: 24.01.2008
(51) Int. Cl.: G01B 11/24, A61C 13/00

(54) **Vorrichtung zur dreidimensionalen optischen Vermessung**
Apparatus for Optical 3D-Measurement
Dispositif de mesure optique en trois dimensions

(30) Priorität: 31.01.2007 DE 102007005726
(43) Veröffentlichungstag der Anmeldung: 30.12.2009
(73) Patentinhaber: Sirona Dental Systems GmbH, 64625 Bensheim (DE)
(72) Erfinder: THIEL, Frank, 64372 Ober Ramstadt (DE); PFEIFFER, Joachim, 64625 Bensheim (DE); FORNOFF, Peter, 64385 Reichelsheim (DE)
(74) Vertreter: Sommer, Peter
(86) Internationale Anmeldenummer: PCT/EP2008/050787
(87) Internationale Veröffentlichungsnummer: WO 2008/092791

(56) Entgegenhaltungen:
- EP-A- 1 797 813
- WO-A-00/08415
- JP-A- 1 313 712
- US-B1- 6 564 087

## Beschreibung

### Technisches Gebiet

Die Erfindung betrifft eine Vorrichtung zur optischen 3D-Vermessung.

### Stand der Technik

Aus dem Stand der Technik sind mehrere Vorrichtung zur optischen 3D-Vermessung bekannt. In vielen Fällen erfolgt die optische 3D-Vermessung durch optische Abtastung der Oberflächen eines Messobjekts mit einem oder mehreren Lichtstrahlen. Die Oberflächen können mit einem punktförmigen Lichtstrahl bzw. mit einem oder mehreren Lichtstreifen abgetastet werden.

Es sind Vorrichtungen bekannt, bei denen eine linienförmige Vermessung durchgeführt wird, indem ein Lichtstreifen in senkrechter Richtung zum Lichtstreifen die Oberfläche abtastet.

Diese Vorrichtungen funktionieren oft auf der Grundlage einer chromatisch konfokalen Messmethode, einer klassischen konfokalen Messmethode mit mechanischen Tiefenscan, einer Triangulation- Messmethode, einer klassischen Interferometrie- Messmethode, einer Weißlichtinterferometrie- Messmethode oder einer Deflektometrie- Messmethode.

Bei der chromatisch konfokalen Messmethode wird eine polychromatische Punktlichtquelle durch ein sogenanntes chromatisches Objektiv wellenlängenabhängig auf ein Messobjekt abgebildet. Das reflektierte Licht wird hinter einer Blende mit Hilfe eines Farbsensors, z.B. eines Spektrometers oder einer CCD- Kamera, spektral analysiert. Aus der Spektralverteilung des zurückgestrahlten Lichts kann die relative Lage des Messobjekts in der z- Richtung senkrecht zur Oberfläche des Messobjekts bestimmt werden.

Bei einer klassischen konfokalen Messmethode mit mechanischen Tiefenscan wird die Fokuslage durch mechanisches Scannen der Optik oder des Objekts relativ zur Optik verändert.

Bei der Triangulation- Messmethode wird ein einzelner Lichtstrahl oder mehrere beispielsweise parallele Lichtstreifen auf das Messobjekt projiziert und senkrecht zur Ausrichtung der Lichtstreifen bewegt. Das reflektierte Licht wird dann mittels eines Bildsensors, wie beispielsweise einer CCD- Kamera, detektiert. Aus der Lage und den Winkeln des Projektionsstrahls und eines zurückgestrahlten Beobachtungsstrahls zum Messobjekt kann unter Verwendung trigonometrischer Rechenmethoden der Abstand zum Messobjekt bestimmt werden.

Bei der klassischen Interferometrie- Messmethode werden mittels eines Interferometers unter Verwendung des physikalischen Effekts der Interferenz Informationen über das Messobjekt gewonnen. Der vom Messobjekt zurückgestrahlte Beobachtungsstrahl wird mit dem Beleuchtungsstrahl mittels eines Interferometers zu einem Interferogramm überlagert. Voraussetzung für eine erfolgreiche Interferenz ist, dass die Lichtstrahlen kohärent überlagert werden. Das bedeutet, dass die aus verschiedenen Richtungen kommenden Lichtstrahlen nur dann interferieren können, wenn sich die Weglängen der beiden Lichtstrahlen nur um weniger als die Kohärenzlänge unterscheiden. Die Kohärenzlänge ist dabei abhängig von der Wellenlänge und der spektralen Bandbreite des verwendeten Lichts.

Die Weißlichtinterferometrie- Messmethode nutzt die Interferenz eines breitbandigen Lichts, wie beispielsweise des Weißlicht. Diese Messmethode vergleicht die Laufzeit des vom Messobjekt zurückgestrahlten Beobachtungsstrahls mittels eines Interferometers, beispielsweise eines Michelson-Interferometers, mit der Laufzeit des Beleuchtungsstrahls mit bekannter optischer Weglänge als Referenz. Die Interferenz der beiden Lichtstrahlen ergibt ein Muster, aus dem man die relative optische Weglänge abgeleitet werden kann.

Bei der Deflektometrie- Messmethode beobachtet man das Bild eines Lichtmusters, wie beispielsweise eines Gitters, in Reflexion über die Oberfläche des Messobjekts. Aus der Deformation des Gitterbildes kann man den lokalen Gradienten der Oberfläche bestimmen und daraus die 3D-Informationen des Messobjekts erzeugen.

Die Abtastbewegung der Lichtstrahlen wird bei bekannten Vorrichtungen beispielsweise durch einen Drehspiegel innerhalb eines Gehäuses der Vorrichtung realisiert. Der Drehspiegel wird um eine Drehachse, die senkrecht zur Richtung des Lichtstrahls verläuft, gekippt und lenkt den Lichtstrahl durch eine oszillierende Bewegung innerhalb einer Winkelbereichs ab. Der oszillierende Lichtstrahl wird dann durch einen am vorderen Ende der Vorrichtung befindlichen fest befestigten Strahlumlenker auf die Oberfläche des Messobjekts umgelenkt.

In der DE 198 29 278 C1 wird eine 3D-Kamera zur Erfassung von Oberflächenstrukturen, insbesondere für zahnmedizinische Zwecke offenbart. Ein Projektionsstrahl wird in seiner Richtung durch eine Blende verändert und durch ein Prisma am vorderen Ende der Vorrichtung auf das Messobjekt umgelenkt. Ein vom Messobjekt reflektierter Beobachtungsstrahl wird durch das Prisma wiederum umgelenkt und zu einem Bildsensor zur Bildanalyse umgeleitet.

Ein Nachteil solcher Vorrichtungen ist dass, ein Spiegel oder ein Prisma zur Umlenkung des Projektionsstrahls bzw. Beleuchtungsstrahls zum Messobjekt und zur Umlenkung des zurückgestrahlten Beobachtungsstrahls zum Bildsensor verwendet wird, der unbeweglich installiert ist. Dadurch geben seine Ausmaße im Wesentlichen die Größe des möglichen Messfeldes solcher Vorrichtungen vor. Das Messfeld ist das Feld, das auf dem Messobjekt mit dem Projektionsstrahl bzw. Beobachtungsstrahl abgetastet wird. Ein Spiegel oder ein Prisma muss nämlich so groß gestaltet sein, dass der Projektionsstrahl bzw. Beobachtungsstrahl auf das Messobjekt innerhalb des Messfeldes umgelenkt werden kann. Die Bauhöhe der Vorrichtungen ist deshalb bei einem größerem Messfeld ebenfalls so groß, dass insbesondere eine 3D-Vermessung von Zähnen innerhalb einer Mundhöhle eines Patienten erschwert wird.

Aus der WO 00/08415 ist eine Vorrichtung zur konfokalen Vermessung der 3D-Struktur eines Objekts bekannt, wobei zur Vermessung eine zweidimensionale. Matrix von Lichtstrahlen auf die Oberfläche des Objekts eingestrahlt wird wird. Dadurch muss nur noch die Tiefe des Objekts abgetastet werden und es ist kein abtasten innerhalb der zu den einfallenden Lichtstrahlen senkrechten Ebene nötig.

Die EP 1 797 813 A1 offenbart eine optische Messvorrichtung zum Vermessen eines Hohlraums, wobei ein Strahlumlenker an einem drehbar und linear bewegbaren Kopfteil, beispielsweise einem Glasstab gehalten wird. So kann mittels der Bewegung des Strahlumlenkers der Umfang und die Tiefe des Hohlraums abgetastet werden.

Aus der US 6,564,087 B1 ist ein Messkopf bekannt, der einen entlang einer Längsachse des Messkopfs beweglichen Strahlumlenker enthält, der einen Projektionsstrahl auf ein Objekt umlenkt. Durch diese Beweglichkeit des Strahlumlenkers kann der Projektionsstrahl auch mittels eines eher kleinen Strahlumlekers über ein möglichst grosses Messfeld bewegt werden. Dadurch kann die Bauhöhe der Vorrichtung möglichst klein gehalten werden, ohne damit das Messfeld einzuschränken.

Aufgrund der Bewegung des Strahlumlenkers entstehen allerdings Beschleunigungskräfte, die Drehmomente verursachen. Solche Drehmomente können zum Vibrieren der ganzen Vorrichtung führen und daher zu einer Verwacklung der Vermessung führen.

Die Aufgabe dieser Erfindung besteht also darin, eine Vorrichtung zur 3D-Vermessung bereitzustellen, die in ihrer Bauhöhe schmal gestaltet ist, die Abtastung eines möglichst großen Messfeldes erlaubt und zugleich verwacklungsfreie Aufnahmen ermöglicht.

### Darstellung der Erfindung

Diese Aufgabe wird durch die vorliegende Erfindung gelöst. Erfindungsgemäß umfasst die Vorrichtung zur optischen 3D-Vermessung einen ersten Strahlumlenker zur Umlenkung eines Beleuchtungsstrahls auf ein Messobjekt und zur Umlenkung des vom Messobjekt zurückgestrahlten Beobachtungsstrahls, wobei der erste Strahlumlenker entlang einer Wegstrecke S bewegbar ist. Weiterhin wird eine Gegenmasse entgegengesetzt zur Bewegung des ersten Strahlumlenkers bewegt, so dass die bei der Bewegung des ersten Strahlumlenkers und der Gegenmasse entstehenden Kräfte und Drehmomente sich nach Möglichkeit gegenseitig aufheben.

Der erste Strahlumlenker kann beispielsweise in Form eines Umlenkspiegels, eines Umlenkprisma oder einer sonstigen

Vorrichtung ausgeführt sein, die sich richtungsändernd auf einen Lichtstrahl auswirkt.

Die Vorrichtung kann für bekannte optischer Messmethoden, wie die chromatisch konfokalen Messmethode, die Triangulation- Messmethode, die klassischen Interferometrie- Messmethode, die Weißlichtinterferometrie- Messmethode oder die Deflektometrie- Messmethode, verwendet werden.

Der Beleuchtungsstrahl kann abhängig von der verwendeten Messmethode sowohl monochromatisch als auch polychromatisch sein. Bei Verwendung eines monochromatischen Beleuchtungsstrahls für die Triangulation- Messmethode, die klassischen Interferometrie- Messmethode, die Weißlichtinterferometrie-Messmethode oder die Deflektometrie- Messmethode muss der Beleuchtungsstrahl nicht notwendigerweise fokussiert werden. Falls keine Fokussierung erfolgt wird ausschließlich der erste Strahlumlenker, ohne ein fokussierendes optisches System, wie ein Objektiv, entlang der Wegstrecke S bewegt. Ansonsten wird der Beleuchtungsstrahls mittels eines fokussierenden optischen Systems, wie eines Objektivs, der mit dem ersten Strahlumlenker mitbewegt wird, fokussiert.

Der sich entlang der Wegstrecke S bewegende erste Strahlumlenker lenkt den Beleuchtungsstrahl auf das Messobjekt, wobei der eingestrahlte Beleuchtungsstrahl unverändert bleibt. Somit erfolgt eine Abtastbewegung des Beleuchtungsstrahls durch die Bewegung des ersten Strahlumlenkers entlang der Wegstrecke S. Der erste Strahlumlenker kann beispielsweise linear entlang einer Wegstrecke S bewegt werden, die parallel zur Richtung des eingestrahlten Beleuchtungsstrahls ist, so dass ein Messfeld auf dem Messobjekt mit der Länge S abgetastet wird. Der Beleuchtungsstrahl trifft auf das Messobjekt und wird durch Reflexions- und Streuvorgänge teilweise zurückgestrahlt und wird als Beobachtungsstrahl durch den ersten Strahlumlenker zurück an den Bildsensor zur Bildsignalanalyse weitergeleitet.

Die 3D-Vermessung eines Messfeldes kann durch einmaliges Abtasten durch den Beobachtungsstrahl erfolgen. Bei einem Beleuchtungsstrahl in Form eines Streifens kann die 3D-Vermessung durch eine einmalige Abtastbewegung in senkrechter Richtung zum Streifen erfolgen. Diese einmalige Abtastbewegung wird dabei durch eine einmalige Bewegung des ersten Strahlumlenkers entlang der Wegstrecke S erzeugt. Als Beleuchtungsstrahl können auch mehrere Lichtstreifen oder auch sonstige Lichtmuster verwendet werden. Ein punktförmiger Beleuchtungsstrahl muss die Abtastbewegung mehrmals vorführen um ein Messfeld zeilenweise abzutasten.

Der erste Strahlumlenker ist im Gegensatz zu bekannten Vorrichtungen, die einen fest montierten Strahlumlenker zur Umlenkung des Beobachtungsstrahls aufweisen, beweglich. Der Beleuchtungsstrahl bleibt dabei in seiner Richtung unverändert. Dadurch muss der erste Strahlumlenker nur von geringen Ausmaßen sein, um den Beobachtungsstrahl umzulenken. Die Vorrichtung kann folglich in ihrer Bauhöhe schmaler als die bekannten Vorrichtungen konstruiert werden, so dass besonders bei zahnmedizinischer Anwendung der Zugang zu Zähnen innerhalb einer Mundhöhle erheblich erleichtert wird.

Ein weiterer Vorteil ist, dass mit der erfindungsgemäßen Vorrichtung ein größeres Messfeld mit der Länge S abgetastet werden kann, denn die Wegstrecke S ist ausschließlich durch die Baulänge der Vorrichtung begrenzt.

Die Gegenmasse ist innerhalb des Gehäuses der Vorrichtung angebracht und wird entgegengesetzt zum ersten Strahlumlenker bewegt. Abhängig von der Beschleunigung und der bewegten Masse entstehen Beschleunigungskräfte, die Drehmomente als Vektorprodukt aus Kraft und Wegstrecke zum Drehpunkt verursachen. Beispielsweise kann die Gegenmasse die gleiche Masse wie der erste Strahlumlenker mit seinen mitbewegten Befestigungsteilen aufweisen, so dass bei entgegengesetzter Bewegung mit Beschleunigungen vom gleichen Betrag entgegengerichtete Beschleunigungskräfte entstehen die sich gegenseitig ausgleichen. Die Beschleunigungskräfte greifen an Punkten an, deren orthogonale Abstände zum Drehpunkt möglichst gleich sind, so dass die Drehmomente sich ebenfalls nahezu ausgleichen. Die Angriffspunkte der Beschleunigungskräfte sind dabei die Massenschwerpunkte der bewegten Massen, nämlich des ersten Strahlumlenkers mit seinen Befestigungsteilen und der Gegenmasse. Durch die Kraftausgleichseinheit wird folglich ein Vibrieren der Vorrichtung während der 3D-Vermessung verhindert, das ansonsten durch den Benutzer kompensiert werden müsste und verwacklungsfreie Vermessung unmöglich machen würde.

Vorteilhafterweise kann der Strahlumlenker entlang der Wegstrecke S oszillierend bewegbar sein und entlang der Wegstrecke S mittels Führungsmittel innerhalb des Gehäuses der Vorrichtung linear geführt werden. Dabei wird die Gegenmasse entgegengesetzt zur linearen Bewegung des ersten Strahlumlenkers mittels Führungsmittel innerhalb des Gehäuses der Vorrichtung linear geführt.

Durch das Führungsmittel wird der erste Strahlumlenker in seinen Freiheitsgraden auf eine lineare Bewegung begrenzt.

Durch das Führungsmittel wird der Strahlumlenker und die Gegenmasse in der gewünschten Richtung und innerhalb der gewünschten Wegstrecke S geführt.

Falls der Beleuchtungsstrahl punktförmig ist, kann dieser bei der 3D-Vermessung das Messfeld zeilenweise oszillierend abtasten. Eine solche Abtastbewegung wird durch die oszillierende Bewegung des Strahlumlenkers entlang der Wegstrecke S erzeugt.

Bei einem Beleuchtungsstrahl in Form eines Streifens reicht eine einmalige Abtastbewegung aus, um ein Messfeld mit der Breite des Streifens zu vermessen. Eine solche 3D-Vermessung kann jedoch auch oszillierend erfolgen, um eine kontinuierliche Abtastung der aktuellen Oberflächeninformation zu erhalten. Dies ist bei sich zeitlich ändernden Messobjekten wesentlich. Bei einer solchen oszillierenden 3D-Vermessung kann die Vorrichtung um das Messobjekt bewegt werden, um Oberflächeninformationen des Messobjekts aus unterschiedlichen Richtungen zu erhalten. Die einzelnen 3D-Aufnahmen aus verschiedenen Richtungen können dann zu einer gesamten 3D-Aufnahme des Messobjekts zusammengesetzt werden.

Die 3D-Vermessung eines Messfeldes kann durch einmaliges Abtasten durch den Beobachtungsstrahl erfolgen. Bei einem Beleuchtungsstrahl beispielsweise in Form eines Streifens kann durch eine einmalige Abtastbewegung in senkrechter Richtung zum Streifen, die durch eine einmalige Bewegung des ersten Strahlumlenkers entlang der Wegstrecke S erzeugt wird, die 3D- Vermessung erfolgen.

Vorteilhafterweise kann ein Objektiv zur Fokussierung des Beleuchtungsstrahls zusammen gleichlaufend mit dem entlang der Wegstrecke S bewegbaren ersten Strahlumlenker bewegbar sein.

Bei der chromatisch konfokalen Messmethode wird der polychromatische Beleuchtungsstrahl auf die Oberfläche des Messobjekts fokussiert, so dass ein Objektiv mit dem ersten Strahlumlenker zusammen gleichlaufend bewegt werden muss, um den Fokuspunkt bei gleichbleibender Brennweite des Objektivs in einer Ebene parallel zur Bewegungsrichtung des ersten Strahlumlenkers zu halten. Bei Verwendung eines polychromatischen Beleuchtungsstrahls für die übrigen optischen 3D-Vermessungsverfahren, wie die Triangulation- Messmethode, die klassischen Interferometrie- Messmethode, die Weißlichtinterferometrie- Messmethode oder die Deflektometrie- Messmethode, muss der Beleuchtungsstrahl auch fokussiert werden, so dass ebenfalls ein mit dem ersten Strahlumlenker mitbewegtes Objektiv notwendig ist.

Vorteilhafterweise kann die Bewegung der Gegenmasse an die Bewegung des ersten Strahlumlenkers über Kopplungsmittel gekoppelt sein. Dadurch muss die Gegenmasse nicht separat angetrieben werden und aufwendig zum Antrieb für die lineare Bewegung des ersten Strahlumlenkers synchronisiert werden. Eine mechanische Kopplung hat nämlich den Vorteil, dass die Bewegungen des ersten Strahlumlenkers und der Gegenmasse bei unvorhergesehener eventueller Frequenzänderung trotzdem synchron bleiben.

Die Kopplungsmittel können beispielsweise Zahnräder, Zahnstangen, Federn und/ oder elastische Fasern sein. Dadurch werden die Bewegungen mechanisch auf eine einfache Weise aneinander gekoppelt.

Die Wegstrecke kann beispielsweise S zwischen 1 mm und 40 mm betragen. Dadurch kann ein Messfeld mit der Länge S zwischen 1 mm und 40 mm abgetastet werden. Bei 3D-Aufnahme in der Mundhöhle eines Patienten können somit Abschnitte einer Zahnreihe vermessen werden.

Die oszillierende Bewegung des ersten Strahlumlenkers kann beispielsweise eine Frequenz zwischen 1 Hz und 100 Hz betragen. Für eine zeitsparende 3D-Vermessung sollte die Frequenz der Abtastbewegung möglichst hoch eingestellt werden. Andererseits ist die Verarbeitungsgeschwindigkeit des Bildsensors, ein begrenzender Faktor für die Frequenz der Abtastbewegung.

Vorteilhafterweise kann der erste Strahlumlenker ein Teil einer Scaneinheit sein, die oszillierend innerhalb des Gehäuses der Vorrichtung bewegbar ist. Der erste Strahlumlenker kann sich beispielsweise innerhalb einer transparenten Scaneinheit aus Plexiglas befinden, die zusammen mit dem ersten Strahlumlenker in Form gegossen wurde. Der erste Strahlumlenker kann auch auf eine andere Weise mit der Scaneinheit fest verbunden sein. Die gesamte Scaneinheit vollführt somit zusammen mit dem Strahlumlenker die oszillierende, vorzugsweise lineare Abtastbewegung innerhalb der Vorrichtung. Die Masse der Gegenmasse muss demnach so ausgelegt sein, dass die Beschleunigungskraft der gesamten Scaneinheit ausgeglichen wird. Beispielsweise kann die Gegenmasse die Masse der Scaneinheit aufweisen. Eine weitere Alternative wäre eine Gegenmasse mit geringerer oder größerer Masse mit einer stärkeren oder schwächeren Beschleunigung, um eine ausgleichende Beschleunigungskraft bereitzustellen.

Vorteilhafterweise kann die Scaneinheit mehrere Strahlumlenker umfassen, um den Beleuchtungsstrahl mehrfach umzulenken. Dadurch kann der Strahlengang des Beleuchtungsstrahls innerhalb des Gehäuses der Vorrichtung in gewünschter Weise umgelenkt werden, so dass die Bauform der Vorrichtung variieren kann.

Vorteilhafterweise kann die Scaneinheit ein Objektiv zur Fokussierung des Beleuchtungsstrahls auf die Oberfläche des Messobjekts umfassen. Das Objektiv wird zusammen mit dem ersten Strahlumlenker in der Scaneinheit bewegt, so dass die parallelen Beleuchtungsstrahlen auf die Oberfläche des Messobjekts fokussiert werden. Die Brennweite des Objektivs entspricht demnach der Wegstrecke von der Hauptebene des Objektiv entlang der optischen Achse zum ersten Strahlumlenker und vom ersten Strahlumlenker bis zum Fokuspunkt auf der Oberfläche des Messobjekts. Die Gegenmasse kann der Masse der Scaneinheit mit dem Objektiv entsprechen, um die Beschleunigungskraft auszugleichen.

Vorteilhafterweise kann das Objektiv ein chromatisches Objektiv sein und der Beleuchtungsstrahl wellenlängenabhängig auf die Oberfläche des Messobjekts fokussiert werden. Bei der chromatisch konfokalen Messmethode zur 3D-Vermessung wird ein chromatischen Objektiv verwendet. Die Brennweite ändert sich abhängig von der Wellenlänge der eingestrahlten Lichtphotonen, so dass nur ein begrenzter Spektralbereich des eingestrahlten polychromatischen Lichts auf der Oberfläche des Messobjekts scharf fokussiert wird. Eine Spektralanalyse des reflektierten Lichts gibt dann Aufschluss über die relative Lage des Messobjekts entlang einer Strahlmittelachse.

Vorteilhafterweise kann die Vorrichtung einen Anschluss für einen Lichtleiter aufweist, der die Vorrichtung mit einer Basiseinheit verbindet, wobei der Lichtleiter den Beleuchtungsstrahl von einer Lichtquelle in der Basiseinheit an die Vorrichtung weiterleitet und den vom Messobjekt zurückgestrahlten Beobachtungsstrahl an die Basiseinheit zurückführt, der mittels eines Strahlteilers umgelenkt und analysiert wird. Dadurch kann die Vorrichtung unabhängig von der Basiseinheit bewegt werden. Die Basiseinheit umfasst dabei die Lichtquelle, den Strahlteiler vor der Lichtquelle und einen Lichtsensor, wie eines Spektrometers oder einer CCD-Kamera, zur Analyse des reflektierten Lichts. Darüber hinaus kann die Vorrichtung in ihren Außenmaßen kleiner und leichter hergestellt werden, so dass der Bedienkomfort und der Zugang zu einem Messobjekt, insbesondere zu Zähnen innerhalb einer Mundhöhle bei zahnärztlicher Anwendung, verbessert wird.

Der erste Strahlumlenkers kann sich beispielsweise an einem gegenüber dem Anschluss für den Lichtleiter liegenden Ende der Vorrichtung befindet. Dadurch wird der Zugang insbesondere zu den hinteren Backenzähnen erleichtert.

Vorteilhafterweise kann der erste Strahlumlenker einen Winkel von 45° zum Beleuchtungsstrahl aufweisen, so dass dieser im rechten Winkel ablenkt wird. Die Vorrichtung muss demnach senkrecht zu der zu vermessenden Oberfläche des Messobjekts gehalten werden. Dadurch wird die optische 3D-Vermessung von Zahnoberflächen verbessert, denn Zahnoberflächen reflektieren den Beleuchtungsstrahl vorwiegend diffus in alle Richtungen oberhalb der Zahnoberfläche mit einer Vorzugsrichtung größter Intensität senkrecht zur Zahnoberfläche.

Vorteilhafterweise kann der Beleuchtungsstrahl aus einer Reihe von Teilstrahlen bestehen, die in einer Ebene angeordnet sind. Dadurch kann eine parallele 3D-Vermessung mit den Teilstrahlen durchgeführt werden, so dass die Vermessungsdauer verkürzt wird. Falls eine chromatisch konfokale Messmethode Anwendung findet, können mehrere in einer Reihe nebeneinander angeordnete punktförmigen Teilstrahlen verwendet werden, die einzeln mittels der im Strahlengang der Teilstrahlen angeordneten chromatische Objektive fokussiert werden und die zurückgestrahlten Beobachtungsstrahlen mittels der entsprechend angeordneten Blenden konfokal auf einen Farbsensor abgebildet werden.

Vorteilhafterweise kann die Vorrichtung zur optischen 3D-Vermessung ein dentales Handstück zur Vermessung von Zahnoberflächen in der Mundhöhle eines Patienten sein. Dadurch kann die erfindungsgemäße Vorrichtung zu zahnärztlichen Zwecken verwendet werden.

Vorteilhafterweise kann der erste Strahlumlenker zwischen zwei Positionen verstellbar sein, wobei der erste Strahlumlenker in der ersten Position den Beleuchtungsstrahl in eine erste Richtung umlenkt und in der zweiten Position den Beleuchtungsstrahl in eine zweite Richtung umlenkt, die zu der ersten Richtung entgegengesetzt ist.

In der ersten Position kann der Beleuchtungsstrahl in die erste Richtung, beispielsweise zu den Zähnen des unteren Kiefers, und in der zweiten Position in die zweite Richtung, beispielsweise zu den Zähnen des oberen Kiefers, umgelenkt werden. Dadurch kann die Richtung der 3D-Vermessung der Vorrichtung gewechselt werden, ohne die Vorrichtung wenden zu müssen. Die Verstellung von der ersten in die zweite Position kann durch eine Drehung des Strahlumlenkers um eine Achse die senkrecht zum Beleuchtungsstrahl ist erfolgen.

Vorteilhafterweise kann der erste Strahlumlenker bei einer ersten Ausrichtung der Polarisation den Beleuchtungsstrahl in die erste Richtung und in einer zweiten Ausrichtung der Polarisation in die zweite Richtung umlenkt, die zu der ersten Richtung entgegengesetzt ist.

Bei einer ersten Ausrichtung der Polarisation wird der linear polarisierte Beleuchtungsstrahl in die erste Richtung und einer zweiten Ausrichtung der Polarisation in die zweite Richtung umgelenkt. Ein solcher polarisationsabhängiger Strahlumlenker kann beispielsweise aus einem Polarisationsfilter und einem im Strahlengang des Beleuchtungsstrahls dahinter angeordneten Umlenkspiegel bestehen. Falls die Polarisation des Beleuchtungsstrahls senkrecht zur Durchlassrichtung des Polarisationsfilters ist, wird der Beleuchtungsstrahl nahezu vollständig vom Polarisationsfilter reflektiert und in die erste Richtung umgelenkt. Falls die Polarisation des Beleuchtungsstrahls parallel zur Durchlassrichtung des Polarisationsfilters ist, wird der Beleuchtungsstrahl nahezu vollständig transmittiert und vom dahinter angeordneten Umlenkspiegel reflektiert und somit in die zweite entgegengesetzte Richtung umgelenkt.

Vorteilhafterweise kann der erste Strahlumlenker in seinem Winkel bezüglich der Ausrichtung des Beleuchtungsstrahls verstellbar sein.

Dadurch kann ein gewünschter Umlenkwinkel des Beleuchtungsstrahls eingestellt werden, so dass auch schwer zugängliche Messfelder, die vor der Vorrichtung liegen, vermessen werden können.

Ein weiterer Gegenstand der vorliegenden Erfindung ist ein Verfahren zur optischen 3D-Vermessung mit einer Vorrichtung, umfassend einen ersten Strahlumlenker, wobei mittels des ersten Strahlumlenkers, der entlang einer Wegstrecke S bewegt wird, ein Beleuchtungsstrahl auf ein Messobjekt umgelenkt wird und ein vom Messobjekt zurückgestrahlter Beobachtungsstrahl erneut umgelenkt wird.

Dabei wird die Gegenmasse einer Kräfteausgleichseinheit entgegengesetzt zum ersten Strahlumlenker oszillierend und/oder linear bewegt, so dass die bei der Bewegung des ersten Strahlumlenkers und der Gegenmasse entstehenden Kräfte und Drehmomente sich nach Möglichkeit gegenseitig aufheben.

Eine Abtastbewegung des Beleuchtungsstrahls erfolgt also durch die Abtastbewegung des ersten Strahlumlenkers, wobei der erste Strahlumlenker beispielsweise linear entlang einer Wegstrecke S bewegt werden kann, die parallel zur Richtung des Beleuchtungsstrahls ist. Auf dem Messobjekt kann dadurch ein Messfeld mit der Länge S vermessen werden.

Die Gegenmasse kann die gleiche Masse wie der erste Strahlumlenker mit seinen mitbewegten Befestigungsteilen aufweisen, so dass bei entgegengesetzter Bewegung sich ausgleichende Beschleunigungskräfte entstehen. Die Beschleunigungskräfte greifen an Punkten der Vorrichtung mit dem gleichen orthogonalen Abstand zu einem Drehpunkt, so dass die Drehmoment sich ebenfalls aufheben. Dadurch verhindert die Kraftausgleichseinheit ein Vibrieren während der 3D-Vermessung, das ansonsten durch den Benutzer kompensiert werden müsste und verwacklungsfreie Vermessung unmöglich machen würde.

Vorteilhafterweise kann die Gegenmasse entgegengesetzt zur Bewegung des ersten Strahlumlenkers linear bewegt werden. Dadurch kann die Gegenmasse entgegengesetzt zur ebenfalls linearen Bewegung des ersten Strahlumlenkers bewegt werden.

Ein Vorteil des Verfahrens ist, dass ein relativ großes Messfeld mit der Länge S abgetastet werden kann, weil der erste Strahlumlenker entlang der Vorrichtung bewegt wird und somit nur durch die Baulänge der Vorrichtung begrenzt ist.

Ein weiterer Vorteil des Verfahrens ist, dass der Beleuchtungsstrahl an jeder Stelle der Messfeldes mit der Länge S unter dem gleichen relativen Winkel zur Vorrichtung auf die Oberfläche des Messobjekts fällt. Dadurch ist die Intensität des reflektierten Strahls besser vergleichbar, denn die Intensität des reflektierten Lichts ist bei diffuser Reflexion winkelabhängig.

Vorteilhafterweise kann der erste Strahlumlenker entlang der Wegstrecke S oszillierend bewegt werden.

Die oszillierende Abtastbewegung, die durch die oszillierende Bewegung des ersten Strahlumlenkers erzeugt wird, ermöglicht eine zeilenweise Abtastung eines Messfeldes mit einem punktförmigen Beleuchtungsstrahl, eine kontinuierliche Abtastung eines sich zeitlich ändernden Messobjekts oder eine Abtastung eines Messobjekts aus unterschiedlichen Richtungen.

Vorteilhafterweise kann der Strahlumlenker entlang der Wegstrecke S linear bewegt werden. Dadurch wird der Beleuchtungsstrahl an jedem Punkt der Wegstrecke S unter dem gleichen Winkel umgelenkt.

Kurzbeschreibung der Zeichnungen Ausführungsbeispiele der Erfindung sind in der Zeichnung dargestellt. Es zeigt die
- Fig. 1: eine Gesamtansicht der Vorrichtung zur 3D- Vermessung, umfassend ein dentales Handstück und eine Basiseinheit, die
- Fig. 2: eine Detailansicht des dentalen Handstücks mit einer Scaneinheit und einer Kraftausgleichsein- heit, die
- Fig. 3: eine Detailansicht des dentalen Handstücks aus Fig. 2 mit Kopplungsmitteln zwischen der Kraft- ausgleichseinheit und der Scaneinheit, die
- Fig. 4: eine Detailansicht der Vorrichtung aus Fig. 1 mit einem ersten Strahlumlenker, der zwischen zwei Positionen verstellbar ist, die
- Fig. 5: eine Detailansicht der Vorrichtung aus Fig. 1 mit einem Strahlumlenker, der abhängig von der Pola- rität den Beleuchtungsstrahl umlenkt, die
- Fig. 6: eine Detailansicht der Vorrichtung aus Fig. 1 mit einem ersten Strahlumlenker, der in seinem Winkel bezüglich der Ausrichtung des Beleuchtungsstrahls verstellbar ist.

### Ausführungsbeispiele der Erfindung

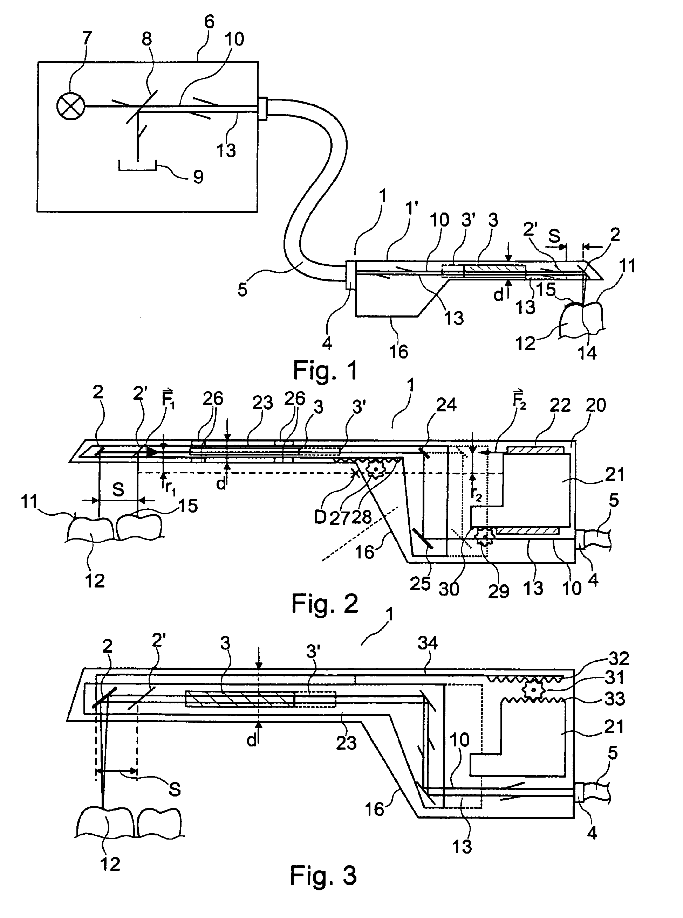

Die Fig. 1 zeigt eine Vorrichtung 1 zur optischen 3D-Vermessung, das einen ersten Strahlumlenker 2 ein Objektiv 3 und einen Anschluss 4 für einen Lichtleiter 5 umfasst. Der erste Strahlumlenker 2 ist dabei entlang einer Wegstrecke S oszillierend bewegbar. Der erste Strahlumlenker 2' nach der linearen Bewegung entlang der Wegstrecke S ist gestrichelt dargestellt. Das Objektiv 3 wird zusammen mit dem ersten Strahlumlenker 2 ebenfalls um die Wegstrecke S linear bewegt. Das gestrichelt dargestellte Objektiv 3' zeigt die Position des Objektivs 3 nach der Bewegung. Die dargestellte Vorrichtung zur optischen 3D-Vermessung 1 hat die Gestalt eines dentalen Handstücks, das über einen Lichtleiter 5 mit einer Basiseinheit 6 verbunden ist. Die Basiseinheit 6 weist eine Lichtquelle 7 einen Strahlteiler 8 und einen Bildsensor 9 auf. Ein eingestrahlter Lichtstrahl 10 wird in der Lichtquelle 7 erzeugt, gelangt abgeschwächt durch den Strahlteiler 8, wird durch den Lichtleiter 5 zum dentalen Handstück 1' geleitet, wird im Handstück 1' durch das Objektiv fokussiert und durch den ersten Strahlumlenker 2 auf die Oberfläche 11 eines Messobjekts 12 umgeleitet. Im vorliegenden Fall ist das Messobjekt ein einzelner Zahn in der Mundhöhle eines Patienten. Der eingestrahlte Lichtstrahl 10 wird von der Zahnoberfläche 11 des Zahns 12 reflektiert. Der diffuse Reflexionsanteil ist dabei höher als der gerichtete Reflexionsanteil. Bei diffuser Reflexion wird das reflektierte Licht in alle Richtungen oberhalb der angestrahlten Fläche abgestrahlt, wobei das Intensitätsmaximum des reflektierten Lichts in senkrechter Richtung zur Fläche abgestrahlt wird. Bei gerichteter Reflexion ist der Einfallswinkel zur Oberfläche gleich dem Ausfallswinkel.

Ein Teil des vorwiegend diffus reflektierten Lichts gelangt somit zurück zum ersten Strahlumlenker und wird von diesem in Form eines Beobachtungsstrahls 13 zum Objektiv 3 umgelenkt. Im Objektiv 3 wird der reflektierte Lichtstrahl 13 wieder in parallel zueinander verlaufende Strahlen umgewandelt und gelangt durch den Lichtleiter 5 zurück zu der Basiseinheit 6. In der Basiseinheit 6 wird ein Teil des Beobachtungsstrahls 13 vom Strahlteiler 8 zum Bildsensor umgeleitet. Das Objektiv 3 fokussiert den einstrahlenden Lichtstrahl 10 auf die Oberfläche 11 des Messobjekts 12, so dass sich der Fokuspunkt 14 des Objektivs 3 auf der Oberfläche 11 befindet. Durch die oszillierende lineare Abtastbewegung des ersten Strahlumlenkers 2 entlang der Wegstrecke S kann ein Messfeld 15 optisch abgetastet werden. Das Messfeld 15 hat demnach aus der Perspektive des Beleuchtungsstrahls 10 eine Länge, die der Wegstrecke S entspricht. Im dargestellten Fall ist die Wegstrecke S 10 mm lang und die Vorrichtung damit geeignet einen einzelnen Zahn zu vermessen. Es können auch Vorrichtungen konstruiert werden deren Mechanik eine Abtastbewegung entlang einer größeren Wegstrecke S erlauben um größere Messflächen zu vermessen. Die Frequenz der Abtastbewegung kann zwischen 1 Hz und 100 Hz gewählt werden. Eine höhere Frequenz würde die Vermessungsdauer verkürzen, darf aber nur so hoch gewählt werden, dass der Bildsensor 9 die einkommenden Bildsignale verarbeiten kann. Die Vorrichtung 1 ist mit einem Gehäuse 16 verkleidet.

Alternativ kann die Vorrichtung als feststehendes oder mobiles Gerät mit allen Bauteilen, umfassend die Lichtquelle und den Bildsensor, in einem Gehäuse ausgeführt sein, wobei ein Datenkabel zur Übertragung der Bilddaten dieses Gerät mit einer Bildanalyseeinheit verbindet.

Der Beleuchtungsstrahl 10 kann die Form eines Lichtpunktes, eines Lichtstreifens, einer Projektion aus mehreren Streifen oder auch andere Formen aufweisen. Der Beleuchtungsstrahl 10 kann abhängig vom verwendeten 3D-Vermessungsverfahren ein monochromatischer oder auch ein polychromatischer Lichtstrahl sein.

Die Fig. 2 zeigt eine Vorrichtung 1 zur optischen 3D-Vermessung aus Fig. 1 mit einer Kraftausgleichseinheit 20. Die Kraftausgleichseinheit 20 umfasst dabei eine Gegenmasse 21 und Führungsmittel 22. Die Gegenmasse 21 wird mittels der Führungsmittel 22 entgegengesetzt zur oszillierenden linearen Abtastbewegung des ersten Strahlumlenkers 2 geführt. Der erste Strahlumlenker 2 ist im Unterschied zu Fig. 1 in einer Scaneinheit 23 befestigt, die auch das Objektiv 3 und zwei weitere Strahlumlenker 24 und 25 umfasst. Der erste Strahlumlenker 2' und das Objektiv 3' sind gestrichelt nach der Abtastbewegung um die Wegstrecke S dargestellt. Die Scaneinheit 23 wird mittels Führungsmittel 26 im dentalen Handstück 1' geführt. Die Gegenmasse 21 hat die gleiche Masse wie die Scaneinheit 23 und wird synchron entgegengesetzt zur Scaneinheit bewegt, so dass Beschleunigungskräfte F₁ der Scaneinheit und F₂ der Gegenmasse 21 sich gegenseitig aufheben. Die Beschleunigungskräfte F₁ und F₂ greifen an Punkten an, deren Abstände r₁ und r₂ zum Drehpunkt D gleich sind, so dass das Drehmoment M₁ als Vektorprodukt aus F₁ und r₁ durch das Drehmoment M₂ als Vektorprodukt aus F₂ und r₂ ausgeglichen wird. Die Kraftausgleichseinheit 20 wird also zu einem Ausgleich der Beschleunigungskraft F₁ und des Drehmoments M₁ verwendet und verhindert dadurch ein Vibrieren der Vorrichtung 1 bei der 3D-Vermessung, das ansonsten durch einen Benutzer kompensiert werden müsste und verwacklungsfreie 3D-Vermessung unmöglich machen würde.

Die Scaneinheit 23 kann beispielsweise mit dem Objektiv 3 und den Strahlumlenkern 2, 24 und 25 als eine Einheit aus transparentem Plexiglas gegossen werden. Die oszillierende lineare Abtastbewegung erfolgt durch Antrieb eines Zahnrades 27, der in eine Zahnstange 28 an der Scaneinrichtung 23 greift. Die entgegengesetzte Bewegung der Gegenmasse 21 wird durch einen weiteren Antrieb eines Zahnrades 29 bewerkstelligt, der in eine Zahnstange 30 an der Gegenmasse 21 greift. Die Antriebe der Zahnräder 27 und 29 werden miteinander synchronisiert, um Bewegungen mit entgegengesetzten Beschleunigungen und Beschleunigungskräften F₁ und F₂ zu gewährleisten. Der einstrahlende Lichtstrahl 10 gelangt wie in Fig. 1 durch den Lichtleiter 5 über den Anschluss 4 in die Vorrichtung 1, wird durch die Strahlumlenker 25 und 24 in der Scaneinheit 23 umgelenkt, wird im Objektiv 3 fokussiert und durch den ersten Strahlumlenker 2 auf das Messobjekt 12 mit der Oberfläche 11 umgelenkt. Der reflektierte Lichtstrahl 13 wird dann wie in Fig. 1 auf dem gleichen Weg zurück zum Bildsensor 9 gelenkt.

Im Vergleich mit bekannten Vorrichtungen ist ein besonderer Vorteil der Vorrichtung 1, dass die Vermessung des Messfeldes 15 mit der Länge S durch die oszillierende lineare Abtastbewegung des ersten Strahlumlenkers 2 erfolgt. Dadurch kann der erste Strahlumlenker 2 mit geringen Ausmaßen konstruiert werden, da er den einstrahlenden Lichtstrahl 10 umlenken muss, der in seiner Ausrichtung während der 3D-Vermessung unverändert bleibt. Folglich kann die Bauhöhe d der Vorrichtung 1 gering gehaltne werden, so dass der Zugang zu Messobjekten, wie Zähne, in der Mundhöhle eines Patienten erleichtert wird. Bei bekannten Vorrichtungen bleibt die Lage des ersten Strahlumlenkers relativ zur Vorrichtung unverändert und die Abtastbewegung erfolgt durch ein Schwenken des Beleuchtungsstrahls 10 durch einen im Strahlengang vor dem ersten Strahlumlenker angebrachten Drehspiegel, so dass die Länge des Messfeldes die Bauhöhe der Vorrichtung maßgeblich bestimmt. Das dargestellte Messobjekt 12 ist in Fig. 1 ein Teil einer Zahnreihe bestehend aus zwei benachbarten Zähnen. Die Vorrichtung 1 aus Fig. 1 und Fig. 2 kann sowohl zur 3D-Vermessung mittels der chromatisch konfokalen Messmethode, als auch mittels der Triangulation-Streifenprojektion- Messmethode oder auch anderer optischer Methoden verwendet werden.

Die Fig. 3 zeigt die Vorrichtung 1 aus Fig. 2, wobei die Bewegungen der Scaneinheit 23 mit dem ersten Strahlumlenker 2 und der Gegenmasse 21 mittels Kupplungsmittel, nämlich eines Antriebszahnrades 31, einer Zahnstange 32 und einer Zahnstange 33, gekoppelt. Die Zahnstange 32 ist über ein Verbindungssteg 34 mit der Scaneinheit 23 verbunden, die den ersten Strahlumlenker 2 umfasst. Die Zahnstange 33 ist an der Gegenmasse 21 angebracht. Bei Antrieb des Zahnrades 31 erfolgt die oszillierende lineare Abtastbewegung der Scaneinheit 23 und die dazu entgegengesetzte Gegenbewegung der Gegenmasse 21, so dass die Kräfte F₁, F₂ und die Drehmomente M₁, M₂ aus Fig. 2 sich gegenseitig aufheben. Der Vorteil der Kopplung im Vergleich zu der Vorrichtung aus Fig. 2 ist, dass die Gegenmasse 21 und die Scaneinheit 23 nicht unabhängig von einander angetrieben werden müssen und die Antriebe der Zahnräder 27 und 29 aufwendig synchronisiert werden müssen.

Die Vorrichtung 1 aus Fig. 3 kann wie die Vorrichtung aus Fig.2 für verschiedene optische 3D-Vermessungsmethoden verwendet werden, die zur Erfassung der Oberflächeninformationen einen oszillierenden Lichtstrahl bzw. Lichtstreifen benötigen. Der einstrahlende Lichtstrahl 10 gelangt wie in Fig. 2 über den Lichtleiter 5 in die Vorrichtung 1 und der reflektierte Lichtstrahl wird mittels des Lichtleiters 5 zurück zu Basiseinheit 6 aus Fig.1 geleitet.

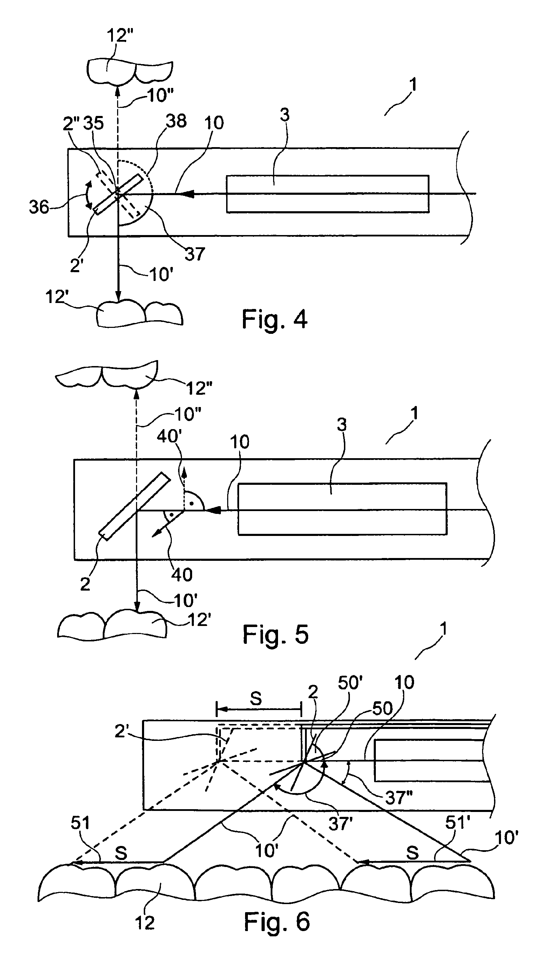

Die Fig. 4 zeigt eine Vorrichtung 1 mit einem ersten Strahlumlenker 2, der zwischen zwei Positionen 2' und 2" um die Achse 35 durch eine Schwenkbewegung 36 verstellbar ist. In der ersten Position 2' lenkt der erste Strahlumlenker 2 in Form eines Umlenkspiegels den Beleuchtungsstrahl 10 in eine erste Richtung 10' zu Zähnen des unteren Kiefers 12' und in der zweiten Position 2" zu Zähnen des oberen Kiefers 12" ab. Somit muss die Vorrichtung 1 nicht gewendet werden, um von der 3D- Vermessung des unteren Kiefers 12' zur 3D-Vermessung des oberen Kiefers 12" zu wechseln. Die erste Richtung 10' weist einen ersten Winkel 37 von 90° und die zweite Richtung 10" einen zweiten Winkel 38 von 90° zum Beleuchtungsstrahl auf. Dieser Winkel 37 kann auch zwischen 5° und 175° betragen.

Die Fig. 5 zeigt eine Vorrichtung 1 mit einem Strahlumlenker 2, der abhängig von der Polarität den Beleuchtungsstrahl 10 entweder in die erste Richtung 10' zu den Zähnen des unteren Kiefers 12' oder in die zweite Richtung 10' zu den Zähnen des oberen Kiefers 12" ablenkt. Bei einer ersten Ausrichtung 40 der Polarisation wird der Beleuchtungsstrahl 10 in die erste Richtung 10' und einer zweiten Ausrichtung 40' der Polarisation in die zweite Richtung 10" umgelenkt. Ein solcher polarisationsabhängiger Strahlumlenker kann beispielsweise aus einem Polarisationsfilter und einem im Strahlengang des Beleuchtungsstrahls 10 dahinter angeordneten Umlenkspiegel bestehen. Falls die Polarisation 40 des Beleuchtungsstrahls 10 senkrecht zur Durchlassrichtung des Polarisationsfilters ist, wird der Beleuchtungsstrahl 10 nahezu vollständig vom Polarisationsfilter reflektiert und in die erste Richtung 10' umgelenkt. Falls die Polarisation 40' des Beleuchtungsstrahls 10 parallel zur Durchlassrichtung des Polarisationsfilters ist, durchstrahlt nahezu vollständig und wird vom dahinter angeordneten Umlenkspiegel reflektiert und in die zweite Richtung 10" umgelenkt.

Die Fig. 6 zeigt eine Vorrichtung 1 mit einem ersten Strahlumlenker 2, der in seinem Winkel 50 bezüglich der Ausrichtung des Beleuchtungsstrahls 10 verstellbar ist. Der erste Strahlumlenker ist in zwei Winkellagen mit einem ersten Winkel 50 von 20° und einem zweiten Winkel 50' von 65° dargestellt. Folglich entsteht in der ersten Winkellage mit dem Winkel 50 von 20° zwischen dem Beleuchtungsstrahl 10 und dem umgelenktem Beleuchtungsstrahl 10' ein Winkel 37' von 140° und in der zweiten Winkellage mit dem Winkel 50' von 65° ein Winkel 37 " von 50°. Die lineare Bewegung des ersten Strahlumlenkers 2 von einer ersten Position 2 längst zur Vorrichtung 1 entlang einer Wegstrecke S zu einer zweiten Position 2' wird in der ersten Winkellage mit dem Winkel 50 wird ein Messfeld 51 mit der Länge S und in der zweiten Winkellage mit dem Winkel 50' wird ein Messfeld 51' abgetastet. Dadurch kann ein Messfeld vor der Vorrichtung 1 abgetastet werden, das nur schwer zugänglich ist, wie beispielsweise im Bereich der molaren Backenzähne.

### Bezugszeichenliste

- 1: Vorrichtung
- 2: erster Strahlumlenker
- 2': erster Strahlumlenker um S bewegt
- 3: Objektiv
- 3': Objektiv um S bewegt
- 4: Anschluss
- 5: Lichtleiter
- 6: Basiseinheit
- 7: Lichtquelle
- 8: Strahlteiler
- 9: Bildsensor
- 10: Beleuchtungsstrahl
- 10': erste Richtung
- 10": zweite Richtung
- 11: Oberfläche, Zahnoberfläche
- 12: Messobjekts, Zahn
- 12': unterer Kiefers
- 12": oberer Kiefers
- 13: Beobachtungsstrahl
- 14: Fokuspunkt
- 15: Messfeld
- 16: Gehäuse
- 20: Kraftausgleichseinheit
- 21: Gegenmasse
- 22: Führungsmittel
- 23: Scaneinheit
- 24: Strahlumlenker
- 25: Strahlumlenker
- 26: Führungsmittel
- 27: Zahnrad
- 28: Zahnstange
- 29: Zahnrad
- 30: Zahnstange
- 31: Antriebszahnrad
- 32: Zahnstange
- 33: Zahnstange
- 34: Verbindungssteg
- 35: Achse
- 36: Schwenkbewegung
- 37: erster Winkel
- 38: zweiter Winkel
- 40: erste Ausrichtung
- 40': zweite Ausrichtung
- 50: Winkel
- 50': zweiter Winkel
- 51: Messfeld
- 51': Messfeld
- S: Wegstrecke
- F₁: Beschleunigungskraft der Scaneinheit
- F₂: Beschleunigungskraft der Gegenmasse
- M₁: Drehmoment der Scaneinheit
- M₂: Drehmoment der Gegenmasse
- D: Drehpunkt
- r₁: Abstand
- r₂: Abstand

## Patentansprüche

1. Vorrichtung (1) zur optischen 3D-Vermessung, umfassend einen ersten Strahlumlenker (2) zur Umlenkung eines Beleuchtungsstrahls (10) auf ein Messobjekt (12) und zur Umlenkung des vom Messobjekt (12) zurückgestrahlten Beobachtungsstrahls (13), wobei der erste Strahlumlenker (2) entlang einer Wegstrecke (S) bewegbar ist, **dadurch gekennzeichnet, dass** eine Gegenmasse (21) einer Kraftausgleichseinheit (20) entgegengesetzt zur Bewegung des ersten Strahlumlenkers (2) bewegt wird, so dass die bei der Bewegung des ersten Strahlumlenkers (2) und der Gegenmasse entstehenden Kräfte (F₁, F₂) und Drehmomente (M₁, M₂) sich nach Möglichkeit gegenseitig aufheben.

2. Vorrichtung (1) nach Anspruch 1, **dadurch gekennzeichnet, dass** der erste Strahlumlenker (2) entlang der Wegstrecke (S) oszillierend bewegbar ist, dass der erste Strahlumlenker (2) entlang der Wegstrecke (S) mittels Führungsmittel (26) innerhalb eines Gehäuses (16) der Vorrichtung (1) linear geführt wird und dass die Gegenmasse (21) entgegengesetzt zur linearen Bewegung des ersten Strahlumlenkers (2) mittels Führungsmittel (22) innerhalb des Gehäuses (16) der Vorrichtung (1) linear geführt wird.

3. Vorrichtung (1) nach Anspruch 1 oder 2, **dadurch gekennzeichnet, dass** ein Objektiv (3) zur Fokussierung des Beleuchtungsstrahls (10) zusammen gleichlaufend mit dem entlang einer Wegstrecke (S) bewegbaren ersten Strahlumlenker (2) bewegbar ist.

4. Vorrichtung (1) nach einem der Ansprüche 1 bis 3, **dadurch gekennzeichnet, dass** die Bewegung der Gegenmasse (21) an die Bewegung des ersten Strahlumlenkers (2) über Kopplungsmittel (31, 32 und 33) gekoppelt ist.

5. Vorrichtung (1) nach einem der Ansprüche 1 bis 4, **dadurch gekennzeichnet, dass** der erste Strahlumlenker (2) ein Teil einer Scaneinheit (23) ist, die oszillierend innerhalb des Gehäuses (16) der Vorrichtung (1) bewegbar ist.

6. Vorrichtung (1) nach einem der Ansprüche 1 bis 5, **dadurch gekennzeichnet, dass** die Scaneinheit (23) mehrere Strahlumlenker (24, 25) umfasst, um den Beleuchtungsstrahl (10) mehrfach umzulenken.

7. Vorrichtung (1) nach einem der Ansprüche 3 bis 6, **dadurch gekennzeichnet, dass** die Scaneinheit (23) das Objektiv (3) zur Fokussierung des Beleuchtungsstrahls (10) auf die Oberfläche (11) des Messobjekts (12) umfasst.

8. Vorrichtung (1) nach einem der Ansprüche 1 bis 7, **dadurch gekennzeichnet, dass** der Beleuchtungsstrahl (10) aus einer Reihe von Teilstrahlen besteht, die in einer Ebene angeordnet sind.

9. Vorrichtung (1) nach einem der Ansprüche 1 bis 8, **dadurch gekennzeichnet, dass** die Vorrichtung (1) zur optischen 3D-Vermessung ein dentales Handstück (1') zur Vermessung von Zahnoberflächen (11) in der Mundhöhle eines Patienten ist.

10. Vorrichtung (1) nach einem der Ansprüche 1 bis 9, **dadurch gekennzeichnet, dass** der erste Strahlumlenker (2) zwischen zwei Positionen (2', 2") verstellbar ist, wobei der erste Strahlumlenker (2) in der ersten Position (2') den Beleuchtungsstrahl (10) in eine erste Richtung (10') umlenkt und in der zweiten Position (2") den Beleuchtungsstrahl (10) in eine zweite Richtung (10") umlenkt, die zu der ersten Richtung (10') entgegengesetzt ist.

11. Vorrichtung (1) nach einem der Ansprüche 1 bis 10, **dadurch gekennzeichnet, dass** der erste Strahlumlenker (2) in seinem Winkel (50, 50') bezüglich der Ausrichtung des Beleuchtungsstrahls (10) verstellbar ist.

12. Vorrichtung (1) nach einem der Ansprüche 1 bis 11, **dadurch gekennzeichnet, dass** der erste Strahlumlenker (2) bei einer ersten Ausrichtung der Polarisation (40) den Beleuchtungsstrahl (10) in die erste Richtung (10') und in einer zweiten Ausrichtung der Polarisation (40') in die zweite Richtung (10") umlenkt, die zu der ersten Richtung (10') entgegengesetzt ist.

13. Vorrichtung (1) nach einem der Ansprüche 3 bis 12, **dadurch gekennzeichnet, dass** das Objektiv (3) ein chromatisches Objektiv ist und der Beleuchtungsstrahl (10) wellenlängenabhängig auf die Oberfläche (11) des Messobjekts (12) fokussiert ist.

14. Vorrichtung (1) nach einem der Ansprüche 1 bis 13, **dadurch gekennzeichnet, dass** die Vorrichtung (1) einen Anschluss (4) für einen Lichtleiter (5) aufweist, der die Vorrichtung (1) mit einer Basiseinheit verbindet, wobei der Lichtleiter (5) den Beleuchtungsstrahl (10) von einer Lichtquelle in der Basiseinheit an die Vorrichtung (1) weiterleitet und den vom Messobjekt (12) zurückgestrahlten Beobachtungsstrahl (13) an die Basiseinheit zurückführt, der mittels eines Strahlteilers umgelenkt und analysiert wird.

15. Verfahren zur optischen 3D-Vermessung mit einer Vorrichtung (1), umfassend einen ersten Strahlumlenker (2), der entlang einer Wegstrecke (S) bewegt wird, einen Beleuchtungsstrahl (10) auf ein Messobjekt (12) umlenkt und einen vom Messobjekt (12) zurückgestrahlten Beobachtungsstrahl (13) zurücklenkt, **dadurch gekennzeichnet, dass** eine Gegenmasse (21) entgegengesetzt zum ersten Strahlumlenker (2) oszillierend und/oder linear bewegt wird, so dass die bei der Bewegung des ersten Strahlumlenkers (2) und der Gegenmasse (21) entstehenden Kräfte und Drehmomente sich nach Möglichkeit gegenseitig aufheben.

## Claims

1. A device (1) for carrying out optical 3D scanning, comprising a first beam deflector (2) for deflecting an illuminating beam (10) onto an object (12) to be scanned and for deflecting the monitoring beam (13) reflected by said object (12), which first beam deflector (2) is movable along a distance (S), **characterized in that** a counterweight (21) pertaining to a force-balancing unit (20) is moved contrarily to the motion of said first beam deflector (2) so that the forces (F₁, F₂) and torques (M₁, M₂) resulting from the motion of said first beam deflector (2) and of said counterweight cancel each other out as far as possible.

2. The device (1) as defined in claim 1, **characterized in that** said first beam deflector (2) can move along distance (S) in an oscillatory manner, that said first beam deflector (2) is linearly guided by guide means (2 6) along said distance (S) in an oscillatory manner within a housing (16) of said device (1), and that said counterweight (21) is linearly guided by guide means (22) within said housing (16) of said device (1) contrary to the linear motion of said first beam deflector (2).

3. The device (1) as defined in claim 1 or claim 2, **characterized in that** an objective (3) for focusing the illuminating beam (10) can be moved concurrently with said first beam deflector (2) as it is moved along said distance (S).

4. The device (1) as defined in any one of claims 1 to 3, **characterized in that** the motion of said counterweight (21) is coupled to the motion of said first beam deflector (2) by coupling means (31, 32, and 33).

5. The device (1) as defined in any one of claims 1 to 4, **characterized in that** said first beam deflector (2) is part of a scanning unit (23) capable of being moved in an oscillatory manner within said housing (16) of said device (1).

6. The device (1) as defined in any one of claims 1 to 5, **characterized in that** said scanning unit (23) comprises a plurality of beam deflectors (24, 25) to enable the illuminating beam (10) to be multiply deflected.

7. The device (1) as defined in any one of claims 3 to 6, **characterized in that** said scanning unit (23) includes said objective (3) for focusing said illuminating beam (10) onto the surface (11) of said object to be scanned (12).

8. The device (1) as defined in any one of claims 1 to 7, **characterized in that** said illuminating beam (10) consists of a series of component beams disposed in a single plane.

9. The device (1) as defined in any one of claims 1 to 8, **characterized in that** said device (1) for carrying out optical 3D scanning is a dental handpiece (1') for scanning tooth surfaces (11) in the oral cavity of a patient.

10. The device (1) as defined in any one of claims 1 to 9, **characterized in that** said first beam deflector (2) can be switched between two positions (2', 2") such that said first beam deflector (2) in the first position (2') deflects the illuminating beam (10) in a first direction (10') and in the second position (2") deflects said illuminating beam (10) in a second direction (10") which is contrary to said first direction (10').

11. The device (1) as defined in any one of claims 1 to 10, **characterized in that** said first beam deflector (2) can be adjusted in terms of its angle (50, 50') to the alignment of said illuminating beam (10).

12. The device (1) as defined in any one of claims 1 to 11, **characterized in that** said first beam deflector (2), in a first direction of polarization (40), deflects said illuminating beam (10) in said first direction (10') and, in a second direction of polarization (40'), deflects said illumination beam (10) in said second direction (10'), which is contrary to said first direction (10').

13. The device (1) as defined in any one of claims 3 to 12, **characterized in that** said objective (3) is a chromatic objective and said illuminating beam (10) is focused wavelength-dependently onto the surface (11) of the object to be scanned (12).

14. The device (1) as defined in any one of claims 1 to 13, **characterized in that** said device (1) has a connector (4) for a light guide(5) that connects said device (1) to a base unit, wherein said light guide (5) passes said illuminating beam (10) from a light source in said base unit to said device (1) and returns the monitoring beam (13) reflected by said object (12) back to said base unit, which monitoring beam is deflected by means of a beam splitter and is analyzed.

15. A method for carrying out optical 3D scanning with a device (1) comprising a first beam deflector (2) that is movable along a distance (S), that deflects an illuminating beam (10) onto an object (12) to be scanned and that returns a monitoring beam (13) reflected by said object (12), **characterized in that** a counterweight (21) is moved contrarily to the motion of said first beam deflector (2) so that the forces and torques resulting from the motion of said first beam deflector (2) and of said counterweight (12) cancel each other out as far as possible.

## Revendications

1. Dispositif (1) pour le mesurage optique en 3D, comprenant un premier déviateur de faisceau (2) pour la déviation d'un faisceau d'éclairage (10) sur un objet mesuré (12) et pour la déviation du faisceau d'observation (13) réfléchi par l'objet mesuré (12), le premier déviateur de faisceau (2) pouvant être déplacé le long d'une section de chemin (S), **caractérisé en ce qu'**une contre-masse (21) d'une unité de compensation de force (20) est déplacée dans le sens opposé au déplacement du premier déviateur de faisceau (2), de sorte que les forces (F₁, F₂) et couples (M₁, M₂), qui se forment lors du déplacement du premier déviateur de faisceau (2) et de la contre-masse, s'annulent éventuellement réciproquement.

2. Dispositif (1) selon la revendication 1, **caractérisé en ce que** le premier déviateur de faisceau (2) peut être déplacé de façon oscillante le long de la section de chemin (S), **en ce que** le premier déviateur de faisceau (2) est guidé de façon linéaire le long de la section de chemin (S) à l'aide de moyens de guidage (26) à l'intérieur d'un boîtier (16) du dispositif (1) et **en ce que** la contre-masse (21) est guidée linéairement dans le sens opposé au déplacement linéaire du premier déviateur de faisceau (2) à l'aide de moyens de guidage (22) à l'intérieur du boîtier (16) du dispositif (1).

3. Dispositif (1) selon la revendication 1 ou 2, **caractérisé en ce qu'**un objectif (3) pour la focalisation du faisceau d'éclairage (10) peut être déplacé de façon synchrone avec le premier déviateur de faisceau (2) mobile le long d'une section de trajet (S).

4. Dispositif (1) selon l'une quelconque des revendications 1 à 3, **caractérisé en ce que** le déplacement de la contre-masse (21) est couplé au déplacement du premier déviateur de faisceau (2) à l'aide de moyen de couplage (31, 32, et 33).

5. Dispositif (1) selon l'une quelconque des revendications 1 à 4, **caractérisé en ce que** le premier déviateur de faisceau (2) est une partie d'une unité de balayage (23), qui peut être déplacée de façon oscillante à l'intérieur du boîtier (16) du dispositif (1).

6. Dispositif (1) selon l'une quelconque des revendications 1 à 5, **caractérisé en ce que** l'unité de balayage (23) comprend plusieurs déviateurs de faisceau (24, 25) pour dévier plusieurs fois le faisceau d'éclairage (10).

7. Dispositif (1) selon l'une quelconque des revendications 3 à 6, **caractérisé en ce que** l'unité de balayage (23) comprend l'objectif (3) pour la focalisation du faisceau d'éclairage (10) sur la surface (11) de l'objet mesuré (12).

8. (Anciennement 18) Dispositif (1) selon l'une quelconque des revendications 1 à 7, **caractérisé en ce que** le faisceau d'éclairage (10) est constitué d'une série de faisceaux partiels qui sont disposés dans un plan.

9. Dispositif (1) selon l'une quelconque des revendications 1 à 8, **caractérisé en ce que** le dispositif (1) pour le mesurage optique en 3D est une pièce à main (1') dentaire pour le mesurage de surfaces de dent (11) dans la cavité buccale d'un patient.

10. Dispositif (1) selon l'une quelconque des revendications 1 à 9, **caractérisé en ce que** le premier déviateur de faisceau (2) peut être réglé déplacé entre deux positions (2', 2"), le premier déviateur de faisceau (2) déviant dans la première position (2') le faisceau d'éclairage (10) dans une première direction (10') et déviant dans la seconde position (2'') le faisceau d'éclairage (10) dans une seconde direction (10"), qui est opposée à la première direction (10').

11. Dispositif (1) selon l'une quelconque des revendications 1 à 10, **caractérisé en ce que** le premier déviateur de faisceau (2) peut être réglé dans son angle (50, 50') par rapport à l'orientation du faisceau d'éclairage (10).

12. Dispositif (1) selon l'une quelconque des revendications 1 à 11, **caractérisé en ce que** le premier déviateur de faisceau (2) dévie dans une première orientation de la polarisation (40) le faisceau d'éclairage (10) dans la première direction (10') et dans une seconde orientation de la polarisation (40') dans la seconde direction (10"), qui est opposée à la première direction (10').

13. Dispositif (1) selon l'une quelconque des revendications 3 à 13, **caractérisé en ce que** l'objectif (3) est un objectif chromatique et le faisceau d'éclairage (10) est concentré en fonction de la longueur d'onde sur la surface (11) de l'objet mesuré (12).

14. Dispositif (1) selon l'une quelconque des revendications 1 à 13, **caractérisé en ce que** le dispositif (1) présente un branchement (4) pour un guide de lumière (5), qui relie le dispositif (1) a une unité de base, le guide de lumière (5) transmettant le faisceau d'éclairage (10) d'une source lumineuse située dans l'unité de base au dispositif (1) et ramenant le faisceau d'observation (13) réfléchi par l'objet à mesurer (12) à l'unité de base, lequel faisceau est dévié au moyen d'un diviseur de faisceau et analysé.

15. Procédé pour le mesurage optique en 3D avec un dispositif (1) comprenant un premier déviateur de faisceau (2), qui est déplacé le long d'une section de chemin (S), dévie un faisceau d'éclairage (10) sur un objet mesuré (12) et renvoie un faisceau d'observation (13) réfléchi par l'objet mesuré (12), **caractérisé en ce qu'**une contre-masse (21) est déplacée de façon oscillante et/ou linéaire dans la direction opposée au premier déviateur de faisceau (2), de sorte que les forces et couples, qui se forment lors du déplacement du premier déviateur de faisceau (2) et de la contre-masse (21), s'annulent éventuellement réciproquement.
